(19) Europäisches Patentamt
European Patent Office
Office européen des brevets

(11) **EP 4 369 354 A1**

(12) **EUROPEAN PATENT APPLICATION**

(43) Date of publication:
**15.05.2024 Bulletin 2024/20**

(21) Application number: **23209046.4**

(22) Date of filing: **10.11.2023**

(51) International Patent Classification (IPC):
**G16H 30/40** (2018.01)      **G06T 7/00** (2017.01)
**G06V 10/82** (2022.01)      **G06V 20/69** (2022.01)
**G16H 50/20** (2018.01)

(52) Cooperative Patent Classification (CPC):
**G16H 30/40; G06T 7/0012; G06V 10/82;**
**G06V 20/69; G16H 10/40; G16H 50/20;**
G06T 2207/20081; G06T 2207/20084;
G06T 2207/30024

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority:  **11.11.2022  KR 20220150893**
          **01.11.2023  KR 20230149215**

(71) Applicant: **Lunit Inc.**
**Seoul 06241 (KR)**

(72) Inventors:
 • **RYU, Jeongun**
  **06241 Seoul (KR)**

 • **SHIN, Jaewoong**
  **06241 Seoul (KR)**
 • **VALERO PUCHE, Aaron**
  **06241 Seoul (KR)**
 • **PARK, Seonwook**
  **06241 Seoul (KR)**
 • **BRATTOLI, Biagio**
  **06241 Seoul (KR)**
 • **PEREIRA, Sérgio**
  **06241 Seoul (KR)**
 • **YOO, Donggeun**
  **06241 Seoul (KR)**
 • **LEE, Jinhee**
  **06241 Seoul (KR)**

(74) Representative: **AWA Sweden AB**
**Box 45086**
**104 30 Stockholm (SE)**

(54)   **METHOD AND APPARATUS FOR ANALYZING PATHOLOGICAL SLIDE IMAGES**

(57)   A computing apparatus includes at least one memory, and at least one processor, wherein the processor is configured to acquire a pathological slide image showing at least one tissue, generate feature information related to at least one area of the pathological slide image, and detect, from the pathological slide image, at least one cell included in the at least one tissue by using the pathological slide image and the feature information.

FIG. 4

START

ACQUIRE PATHOLOGICAL SLIDE IMAGE SHOWING AT LEAST ONE TISSUE — 410

GENERATE FEATURE INFORMATION RELATED TO AT LEAST ONE AREA OF PATHOLOGICAL SLIDE IMAGE — 420

DETECT, FROM PATHOLOGICAL SLIDE IMAGE, AT LEAST ONE CELL INCLUDED IN AT LEAST ONE TISSUE BY USING PATHOLOGICAL SLIDE IMAGE AND FEATURE INFORMATION — 430

END

EP 4 369 354 A1

## Description

BACKGROUND

1. Field

The disclosure relates to a method and apparatus for analyzing a pathological slide image.

2. Description of the Related Art

The field of digital pathology refers to a field of acquiring histological information or predicting a prognosis of a patient by using a whole slide image generated by scanning a pathological slide image.

Recently, technologies have been developed to predict medical information regarding subjects by analyzing pathological slide images via machine learning models. In general, separate machine learning models are respectively used to identify tissues and cells appearing in pathological slide images. However, due to cases where machine learning models do not accurately determine types of cells, the accuracy of analysis of pathological slide images may be lowered.

SUMMARY

Provided are a method and apparatus for analyzing a pathological slide image.

Provided is a computer-readable recording medium having recorded thereon a program for executing the method on a computer.

Technical problems to be solved are not limited to the technical problems as described above, and other technical problems may be present.

Additional aspects will be set forth in part in the description which follows and, in part, will be apparent from the description, or may be learned by practice of the presented embodiments of the disclosure.

According to an aspect of the disclosure, a computing apparatus includes at least one memory, and at least one processor, wherein the processor is configured to acquire a pathological slide image showing at least one tissue, generate feature information related to at least one area of the pathological slide image, and detect, from the pathological slide image, at least one cell included in the at least one tissue by using the pathological slide image and the feature information.

According to another aspect of the disclosure, a method of analyzing a pathological slide image includes acquiring a pathological slide image showing at least one tissue, generating feature information related to at least one area of the pathological slide image, and detecting, from the pathological slide image, at least one cell included in the at least one tissue by using the pathological slide image and the feature information.

According to another aspect of the disclosure, a computer-readable recording medium includes a recording medium having recorded thereon a program for executing the above-described method on a computer.

BRIEF DESCRIPTION OF THE DRAWINGS

The above and other aspects, features, and advantages of certain embodiments of the disclosure will be more apparent from the following description taken in conjunction with the accompanying drawings, in which:

FIG. 1 is a view illustrating an example of a computing apparatus for analyzing a pathological slide image, according to an embodiment;
FIGS. 2A and 2B are views illustrating a general example in which a pathological slide image is analyzed;
FIG. 3 is a block diagram illustrating an example of a computing apparatus, according to an embodiment;
FIG. 4 is a flowchart illustrating an example of a method of analyzing a pathological slide image, according to an embodiment;
FIG. 5 is a view illustrating an example in which a processor detects a cell from a pathological slide image, according to an embodiment;
FIG. 6 is a view illustrating another example in which a processor detects a cell from a pathological slide image, according to an embodiment;
FIG. 7 is a view illustrating another example in which a processor detects a cell from a pathological slide image, according to an embodiment;
FIGS. 8A and 8B are views illustrating an example in which a feature map of a tissue and a feature map of a cell are arranged in the same dimension, according to an embodiment;
FIGS. 9A to 9C are views illustrating examples of training data of a first machine learning model and/or a second machine learning model, according to an embodiment;
FIG. 10 is a view illustrating another example in which a processor detects a cell from a pathological slide image, according to an embodiment;
FIG. 11 is a view illustrating another example in which a processor detects a cell from a pathological slide image, according to an embodiment;
FIG. 12 is a view illustrating an example in which a processor predicts various types of information from a pathological slide image, according to an embodiment; and
FIG. 13 is a view illustrating an example of a system for analyzing a pathological slide image.

DETAILED DESCRIPTION

Terms used in embodiments are selected as currently widely used general terms as possible, which may vary depending on intentions or precedents of one of ordinary skill in the art, emergence of new technologies, and the like. In addition, in certain cases, there are also terms arbitrarily selected by the applicant, and in this case, the meaning thereof will be defined in detail in the description. Therefore, the terms used herein should be defined based on the meanings of the terms and the details throughout the description, rather than the simple names of the terms.

Throughout the description, when a part includes a certain element, it means that other elements may be further included, rather than excluding the other elements, unless otherwise stated.

Also, although the terms, "first", "second", etc. may be used herein to describe various elements, these elements should not be limited by these terms. These terms may be only used to distinguish one element from another.

Hereinafter, a "subject" may refer to a protein, cell, tissue, structure, or the like included in a pathological slide image (or expressed in a pathological slide image).

Hereinafter, a "pathological slide image" may refer to an image acquired by scanning a pathological slide. Here, the pathological slide may be produced by arranging, on a glass slide, a slice obtained by cutting a tissue block collected from a human body into a thin layer form. For example, the pathological slide may be produced by fixing and staining the slice through a series of chemical treatment processes.

The pathological slide image may refer to a whole slide image (WSI) including a high-resolution image of a whole pathological slide, or may refer to a portion (e.g., one or more patches or tiles) of the whole slide image. For example, the pathological slide image may be a digital image scanned via a scanning apparatus (e.g., a digital scanner or the like).

The pathological slide image may be divided into a tissue area in which a tissue collected from a human body is located and a background area, and information regarding a particular protein, cell, tissue and/or structure may be acquired from the tissue area.

Histological information may be applied (e.g., tagged) to one or more patches included in the pathological slide image via an annotation operation.

Hereinafter, "medical information" may refer to any medically meaningful information that may be extracted from a medical image (e.g., a pathological slide image). For example, the medical information may include at least one of an immune phenotype, a genotype, a biomarker score, a tumor purity, information regarding ribonucleic acid (RNA), information regarding a tumor microenvironment, and a treatment method for cancer expressed in a pathological slide image.

In addition, the medical information may include, for a particular tissue (e.g., a cancer tissue, a cancer stroma tissue, a necrosis tissue, or the like) and/or a particular cell (e.g., a tumor cell, a lymphocyte cell, a macrophage cell, an endothelial cell, a fibroblast cell, or the like) within the medical image, areas, locations, sizes, cancer diagnostic information, information associated with the likelihood of developing cancer of a subject, and/or a medical conclusion associated with cancer treatment, but is not limited thereto.

Also, the medical information may include not only a quantified numerical value that may be obtained from the medical image, but also information obtained by visualizing the numerical value, prediction information according to the numerical value, image information, statistical information, and the like.

Hereinafter, the term "extraction" may indicate identifying the presence of a subject in a pathological slide image, regardless of the type of subject. Meanwhile, the term "detection" may indicate not only identifying the presence of a subject but also confirming the type of identified subject.

Embodiments are described in detail below with reference to the attached drawings. However, the embodiments may be implemented in various different forms and are not limited to examples described herein.

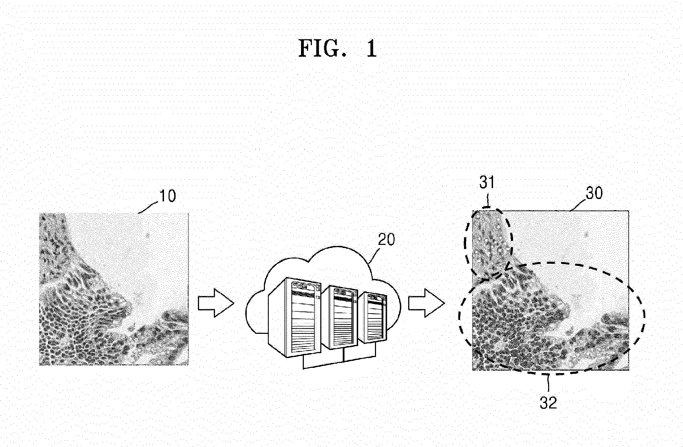

FIG. 1 is a view illustrating an example of a computing apparatus for analyzing a pathological slide image, according to an embodiment.

FIG. 1 illustrates a pathological slide image 10, a computing apparatus 20, and an image 30 in which a result of analysis of the pathological slide image 10 appears. The pathological slide image 10 may show at least one tissue, and the tissue may include at least one cell. Accordingly, various types of medical information as well as information regarding a type of corresponding tissue and/or cell may be generated by analyzing the pathological slide image 10.

The pathological slide image 10 may be a whole slide image or a portion of the whole slide image. For example, the pathological slide image 10 may refer to a patch which is divided from the whole slide image. In detail, the patch may refer to an image which is divided from the whole slide image to have a certain size. In some embodiments, the patch may refer to an area including each of subjects from among the whole slide image.

The computing apparatus 20 may analyze the pathological slide image 10. For example, the computing apparatus 20 may generate various types of information by analyzing the pathological slide image 10 by using at least one machine learning model. Biological factors (e.g., a cancer cell, an immune cell, a cancer area, and the like), which are expressed in the pathological slide image 10 by the analysis of the pathological slide image 10, may be identified. The biological factors may be used for a histological diagnosis of a disease, prediction of a prognosis of the disease, a determination of a treatment direction for the disease, and the like.

For example, the computing apparatus 20 may be an apparatus that includes a memory and a processor, and has a computation capability. In addition, the computing apparatus 20 may be an apparatus that communicates with an external device (not shown) including a user terminal. For example, the computing apparatus 20 may store various types of data, including the pathological slide image 10, a bitmap image corresponding to the pathological slide image 10, information generated by the analysis of the pathological slide image 10, and information regarding a machine learning model used for the analysis of the pathological slide image 10. For example, the computing apparatus 20 may be a cloud server, but is not limited thereto. The computing apparatus 20 may be a notebook PC, a desktop PC, a laptop, a tablet computer, a smart phone, or the like, but is not limited thereto.

When the pathological slide image 10 is analyzed by a machine learning model, in general, a machine learning model (hereinafter, referred to as a tissue identification model) that identifies a tissue in the pathological slide image 10 and a machine learning model (hereinafter, referred to as a cell identification model) that identifies a cell in the pathological slide image 10 may be often different from each other.

Detailed morphological characteristics of a cell, such as a color, opacity, and shape, need to be considered to increase a probability of the cell identified in the pathological slide image 10. Accordingly, a general cell identification model may analyze the pathological slide image 10 by magnifying the pathological slide image 10 at a high magnification. Here, an image magnified at a high magnification may show a relatively small field of view (FoV).

Accordingly, the cell identification model may output a result without considering whole context of the pathological slide image 10. In this case, the cell identification model may not accurately classify and detect a type of cell in the pathological slide image 10.

For example, the whole context may include information regarding organization of the cell. In other words, the whole context may increase a degree of understanding of how cells appearing in the pathological slide image 10 are arranged and grouped to form a higher level structure.

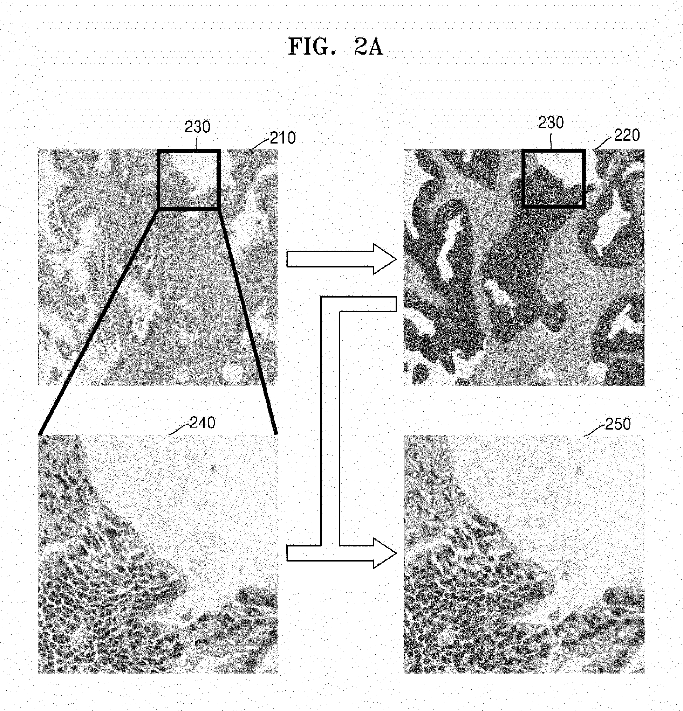

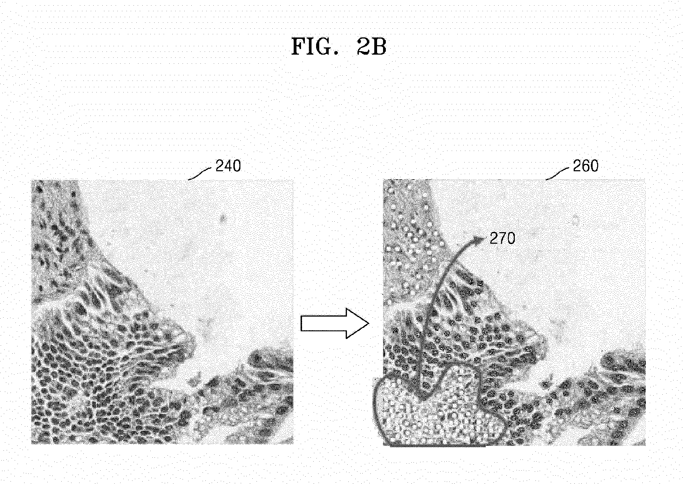

An example in which whole context is considered in analysis of the pathological slide image 10 is described below with reference to FIGS. 2A and 2B.

FIGS. 2A and 2B are views illustrating a general example in which a pathological slide image is analyzed.

FIG. 2A illustrates an example in which whole context is considered when an expert (e.g., a pathologist) analyzes a partial area 230 within a pathological slide image 210 in a real environment. Meanwhile, FIG. 2B illustrates an example in which the whole context is not considered when a cell identification model analyzes the partial area 230 within the pathological slide image 210. An area 240 in FIGS. 2A and 2B may refer to an area obtained by magnifying the area 230 in FIG. 2A at a high magnification.

Referring to FIG. 2A, the expert may first understand the whole context by observing the pathological slide image 210 with a wide field of view (i.e., at a low magnification). Accordingly, the expert may identify an area corresponding to tissues and a background area in the pathological slide image 210. In more detail, the expert may identify a type of each of the tissues (e.g., a cancer tissue, a cancer stromal tissue, a necrosis tissue, and the like) in the area corresponding to the tissues. For example, an image 220 may be a result of observing the pathological slide image 210 with a wide field of view, and each of colors displayed in the image 220 may refer to a particular type of tissue and/or a background area within the image 210.

Subsequently, the expert may identify cells within the area 230 by observing the area 230. In detail, the expert may identify the cells included in the area 230 by magnifying and observing the area 230 (i.e., observing the area 240) with a narrow field of view (i.e., at a high magnification).

Here, the expert may consider the whole context when observing the area 240. In other words, the expert may observe the area 240 by considering what type of tissue is included within the area 240 and/or whether or not a background area is included within the area 240. Accordingly, the expert may not only identify how many cells are present within the area 240, but also identify information regarding what types (e.g., a tumor cell, a lymphocyte cell, a macrophage cell, an endothelial cell, a fibroblast cell, a background cell, and the like) the cells within the area 240 are. For example, an image 250 may be a result of observing the area 240, and differently colored dots displayed in the image 250 may respectively refer to a tumor cell and a background cell.

In other words, in a real environment, the expert may observe the pathological slide image 210 with a wide field of view to understand the whole context of the pathological slide image 210, and then observe the area 230 with a narrow field of view (i.e., observe the area 240) to identify individual cells within the area 230. Accordingly, not only whether or not cells are present within the area 230 but also the types of cells may be accurately identified.

As described above with reference to FIG. 2A, the expert may identify cells within the partial area 230 by considering the whole context of the pathological slide image 210.

In contrast, referring to FIG. 2B, a general cell identification model may identify cells on the basis of a result of observing the area 230 (i.e., observing the area 240) with a narrow field of view, and thus may fail to consider the whole context. In other words, the general cell identification model may analyze the area 240 by considering only detailed morphological characteristics of a cell. Therefore, the general cell identification model may inaccurately detect the type of cell. For example, as illustrated in FIG. 2A, when the image 250 is a ground truth as a result of the expert analyzing the area 240 by considering the whole context, an image 260 may be a result of analyzing the area 240 via a general cell identification model. Here, comparing the image 260 to the image 250, the image 260 may include a result 270 in which a type of cell is inaccurately detected.

Therefore, the disclosure provides a method and computing apparatus for analyzing a pathological slide image by considering context of a whole slide image to increase the accuracy of analysis of the pathological slide image by operating similarly to a process of analyzing a pathological slide image by an actual person.

Referring back to FIG. 1, the computing apparatus 20 according to an embodiment may detect at least one cell within the pathological slide image 10 by analyzing the pathological slide image 10. In other words, the computing apparatus 20 may output results 31 and 32 of detecting cells within the pathological slide image 10 by type.

For example, the computing apparatus 20 may accurately detect at least one cell within the pathological slide image 10 by combining a cell extraction result of analyzing the pathological slide image 10 at a high magnification (i.e., with a narrow field of view) with a tissue division result of analyzing the pathological slide image 10 at a low magnification (i.e., a wide field of view). In other words, the computing apparatus 20 may output the same result as the analysis by the expert described above with reference to FIG. 2A.

Hereinafter, a high-magnification image may refer to an image (e.g., an image including 1,024 * 1,024 pixels) obtained by magnifying a whole slide image at a magnification high enough to analyze cells by using a machine learning model, a lowmagnification image may refer to an image obtained by magnifying a whole slide image at a magnification low enough to analyze whole context by using a machine learning model, or to an original whole slide image (e.g., an image including 4,096 * 4,096 pixels), and a resolution may be 0.2 (micron-per-pixel) MPP, but the disclosure is not limited thereto. In other words, a high magnification and a low magnification may be relative differences, and a quantitative numerical value of a magnification may be diverse.

For convenience of description, hereinafter, the computing apparatus 20 is described as detecting at least one tissue appearing in the pathological slide image 10 (i.e., classifying tissues by type) by using a first machine learning model and detecting at least one cell appearing in the pathological slide image 10 by using a second machine learning model. However, the first machine learning model and the second machine learning model may be the same machine learning model or may be separate machine learning models.

Here, a machine learning model may refer to a statistical learning algorithm implemented on the basis of a structure of a biological neural network, or a structure that executes the statistical learning algorithm. For example, the machine learning model may refer to a model having a problem-solving capability, in which nodes, which are artificial neurons that form a network through a combination of synapses as in a biological neural network, are trained to reduce an error between a correct output corresponding to a particular input and an inferred output by repeatedly adjusting weights of the synapses.

For example, the machine learning model may include a random probability model, a neural network model, or the like used in an artificial intelligence learning method such as deep learning.

For example, the machine learning model may be implemented as a multilayer perceptron (MLP) including multilayer nodes and connections between the multilayer nodes. The machine learning model according to an embodiment may be implemented by using one of various types of artificial neural network model structures including MLPs. For example, the machine learning model may include an input layer that receives an input signal or data from the outside, an output layer that outputs an output signal or data corresponding to the input data, and at least one hidden layer that is located between the input layer and the output layer, receives a signal from the input layer, extracts features, and delivers the extracted features to the output layer. The output layer may receive a signal or data from the hidden layer and output the received signal or data to the outside.

Accordingly, the machine learning model may be trained to receive a pathological slide image to not only detect a subject included in the pathological slide image but also generate information regarding the subject and/or expression information of a biomarker.

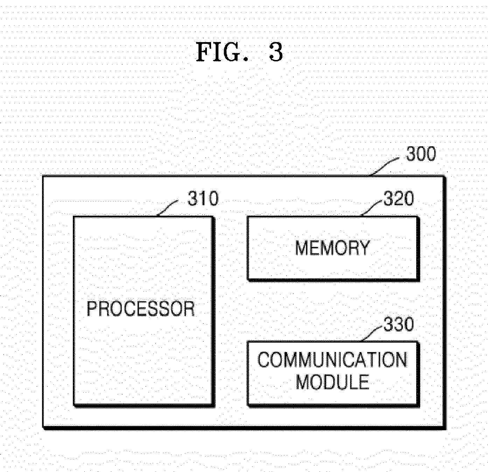

FIG. 3 is a block diagram illustrating an example of a computing apparatus, according to an embodiment.

Referring to FIG. 3, a computing apparatus 300 may include a processor 310, a memory 320, and a communication module 330. For convenience of description, FIG. 3 illustrates only components related to the disclosure. Accordingly, the computing apparatus 300 may further include other general-purpose components, in addition to the components illustrated in FIG. 3. In addition, it is obvious to one of ordinary skill in the art related to the disclosure that the processor 310, memory 320, and communication module 330 illustrated in FIG. 3 may be implemented as independent devices.

The processor 310 may process instructions of a computer program by performing basic arithmetic, logic, and input/output operations. Here, the instructions may be provided from the memory 320 or an external device (e.g., a user terminal or the like). In addition, the processor 310 may generally control operations of other components included in the computing apparatus 300.

The processor 310 may acquire a pathological slide image showing at least one tissue. Also, according to various embodiments, the processor 310 may generate feature information related to at least one area of the pathological slide image. In addition, according to various embodiments, the processor 310 may detect at least one cell included in the at least one tissue from the pathological slide image by using the pathological slide image and the feature information.

According to a first embodiment, the processor 310 may generate, as feature information, a first image showing a first type of area of the pathological slide image by using a first machine learning model. In some embodiments, the processor 310 may generate, as feature information, a first image in which areas of the pathological slide image are classified by type, by using the first machine learning model. Here, the type may include at least one of a cancer area, a cancer stroma area, a necrosis area, and a background area. Also, the first type may include any one of the types described above.

In addition, the processor 310 may detects a second type of cell from the pathological slide image by using a second machine learning model, and may exclude, from a detection result, at least one cell, which is not included in the first type of area, from among the detected cells. In some embodiments, the processor 310 may detect at least one cell on the basis of the pathological slide image and the first image by using the second machine learning model. As an example, the processor 310 may detect at least one cell from the pathological slide image, on the basis of a third image in which the first image and the second image are merged. Here, the second image may refer to an image acquired from a portion corresponding to the first image within the pathological slide image. As another example, the processor 310 may detect at least one cell from the pathological slide image by using the first image in at least one of intermediate operations of the second machine learning model that uses the pathological slide image as an input. As another example, the processor 310 may detect at least one cell from the pathological slide image by mutually using information generated from at least one of intermediate operations of the first machine learning model and information generated from at least one of intermediate operations of the second machine learning model in at least one of the intermediate operations of the first machine learning model and at least one of the intermediate operations of the second machine learning model.

Here, at least one of the first machine learning model and the second machine learning model may be trained by data generated on the basis of at least one patch included in the pathological slide image. For example, training data may include at least one of a first patch magnified at a first magnification, at least one annotated tissue based on the first patch, a second patch magnified at a second magnification, at least one annotated cell based on the second patch, and information regarding a positional relationship between the first patch and the second patch.

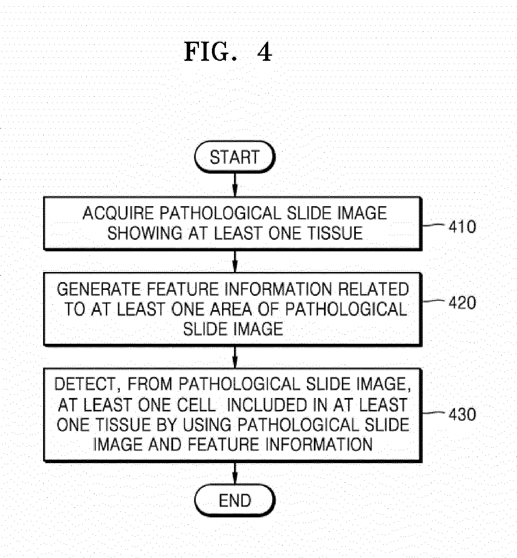

The operation of the processor 310 according to the first embodiment is described in detail with reference to FIGS. 4 to 9.

According to a second embodiment, the processor 310 may generate feature information by analyzing, in a sliding window method, a pathological slide image magnified at a third magnification. Also, the processor 310 may extract cells from a pathological slide image by analyzing, in a sliding window method, the pathological slide image magnified at a fourth magnification. In addition, the processor 310 may detect at least one cell from the pathological slide image by using the result of extraction and the feature information.

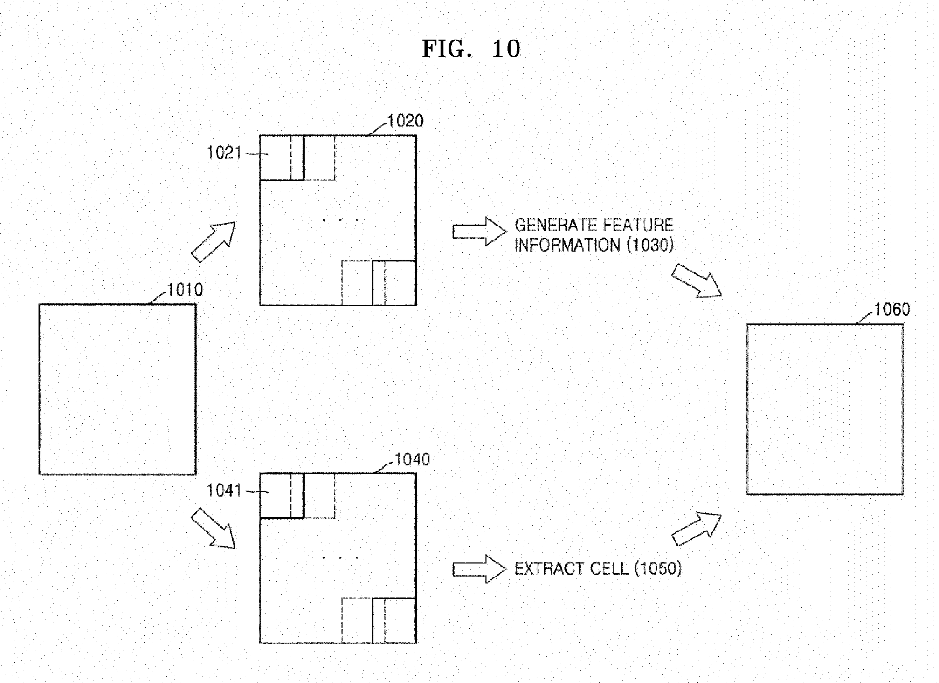

The operation of the processor 310 according to the second embodiment is described in detail with reference to FIGS. 4 and 10.

According to a third embodiment, the processor 310 may detect at least one cell from a pathological slide image by using a third machine learning model. Here, a magnification of an image used for training of the third machine learning model and a magnification of an image used for inference by the third machine learning model may be different from each other.

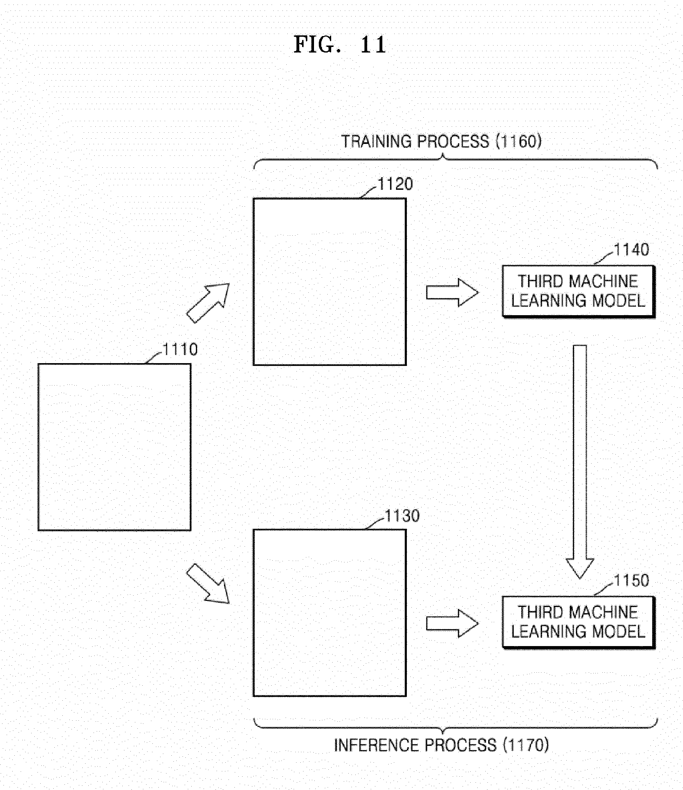

The operation of the processor 310 according to the third embodiment is described in detail with reference to FIGS. 4 and 11.

According to a fourth embodiment, the processor 310 may generate feature information from a pathological slide image. Here, the feature information may include at least one of a cancer area, a tumor cell density, and information regarding a biomarker score. In addition, the processor 310 may further detect, from the pathological slide image, at least one piece of biomarker information, immune phenotype information, lesion information, genomic mutation information, and genomic signature information which are expressed on at least one tissue.

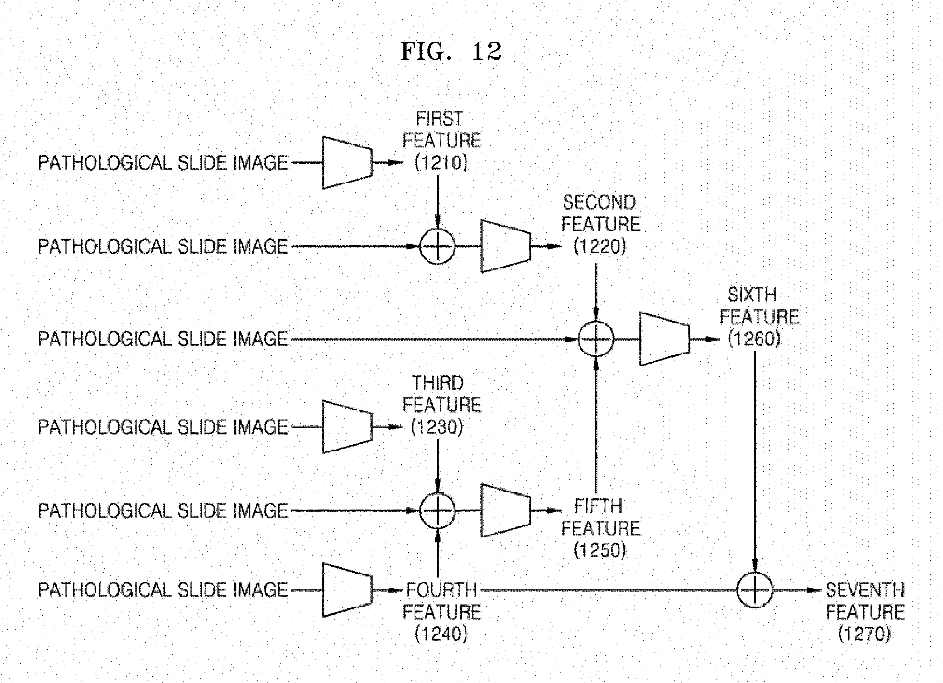

The operation of the processor 310 according to the fourth embodiment is described in detail with reference to FIGS. 4 and 12.

For example, the processor 310 may be implemented as an array of multiple logic gates, or may be implemented as a combination of a general-purpose microprocessor and a memory storing a program that may be executed on the general-purpose microprocessor. For example, the processor 310 may include a general-purpose processor, a central processing unit (CPU), a microprocessor, a digital signal processor (DSP), a controller, a microcontroller, a state machine, or the like. In some environments, the processor 310 may also include an application-specific integrated circuit (ASIC), a programmable logic device (PLD), a field programmable gate array (FPGA), or the like. For example, the processor 310 may refer to a combination of processing devices such as a combination of a digital signal processor (DSP) and a microprocessor, a combination of a plurality of microprocessors, a combination of one or more microprocessors combined with a digital signal processor (DSP) core, or a combination of any other components.

The memory 320 may include any non-transitory computer-readable recording medium. As an example, the memory 320 may include a permanent mass storage device such as random access memory (RAM), read only memory (ROM), a disk drive, a solid state drive (SSD), or flash memory. As another example, the permanent mass storage device, such as ROM, an SSD, flash memory, or a disk drive, may be a separate permanent storage device distinct from a memory. Also, the memory 320 may store an operating system (OS) and at least one program code (e.g., code for the processor 310 to perform an operation described below with reference to FIGS. 4 to 12).

Software components as described above may be loaded from a computer-readable recording medium separate from the memory 320. The separate computer-readable recording medium may be a recording medium that may be directly connected to the computing apparatus 300, and may include, for example, a floppy drive, a disk, a tape, a DVD/CD-ROM drive, a memory card, and the like. In some embodiments, the software components may be loaded into the memory 320 through the communication module 330 rather than through the computer-readable recording medium. For example, at least one program may be loaded into the memory 320 on the basis of a computer program installed by files which are provided, via the communication module 330, by developers or a file distribution system that distributes an installation file of an application (e.g., a computer program for the processor 310 to perform an operation described below with reference to FIGS. 3 to 10, or the like).

The communication module 330 may provide a component or function via which the computing apparatus 300 and an external device communicate with each other through a network. For example, a control signal, a command, data, or the like provided under control of the processor 310 may be transmitted to the external device through the communication module 330 and the network.

Meanwhile, although not shown in FIG. 3, the computing apparatus 300 may further include an input/output interface. For example, the input/output interface may be a unit for interfacing with a device (e.g., a keyboard, a mouse, or the like) for an input or output, which may be connected to the computing apparatus 300 or included in the computing apparatus 300.

Also, although not shown in FIG. 3, the computing apparatus 300 may further include a display apparatus. In some embodiments, the computing apparatus 300 may be connected to an independent display apparatus in a wired or wireless communication method to transmit and receive data to and from each other. For example, a pathological slide image, analysis information of the pathological slide image, medical information, additional information based on the medical information, and the like may be provided to a user through the display apparatus.

FIG. 4 is a flowchart illustrating an example of a method of analyzing a pathological slide image, according to an embodiment.

The method illustrated in FIG. 4 may include operations processed in time series by the computing apparatus 20 or 300 shown in FIG. 1 or 3 or by the processor 310 shown in FIG. 3. Therefore, even when the above description of the computing apparatuses 20 and 300 or the processor 310 is omitted below, the above description may also be applied to the method shown in FIG. 4.

In operation 410, the processor 310 may acquire a pathological slide image showing at least one tissue.

For example, the processor 310 may read a pathological slide image stored in the memory 320. In some embodiments, the processor 310 may receive a pathological slide image from an external device through the communication module 330. For example, the external device may include a user terminal, a scanning apparatus, a server, or the like.

For example, the pathological slide image may be a whole slide image or a portion of the whole slide image. Here, the portion may be referred to as a patch or a tile, and may refer to a partial image of the whole slide image. For example, the patch or the tile may be an image showing a certain subject, or may be an image showing a region of interest defined by a user.

In operation 420, the processor 310 may generate feature information related to at least one area of the pathological slide image.

As an example, the processor 310 may generate, as feature information by using a first machine learning model, a first image showing a first type of area of the pathological slide image. For example, the feature information may include information regarding types of areas of the pathological slide image. Also, the feature information may be expressed on the first image.

Here, the first type of area may be a cancer area within the pathological slide image, but is not limited thereto. For example, the first type of area may be any one of a cancer area, a cancer stroma area, a necrosis area, and a background area.

For example, the processor 310 may output, by using the first machine learning model, a detection result in the form of layers showing tissues on the pathological slide image. In this case, the first machine learning model may be trained to detect, within the pathological slide image, areas corresponding to tissues within a plurality of reference pathological slide images by using training data including the plurality of reference pathological slide images and a plurality of pieces of reference label information.

Also, the processor 310 may classify a plurality of tissues expressed in the pathological slide image. In detail, the processor 310 may classify the pathological slide image into at least one of a cancer area, a cancer stroma area, a necrosis area, and a background area.

However, the example in which the processor 310 classifies at least partial area expressed in the pathological slide image is not limited to the above description. In other words, without being limited to four types of areas described above (e.g., a cancer area, a cancer stroma area, a necrosis area, and a background area), the processor 310 may classify at least one area expressed in the pathological slide image into a plurality of categories, according to various criteria. The at least one area expressed in the pathological slide image may be classified into the plurality of categories according to preset criteria or criteria set by the user.

As another example, the processor 310 may generate, as feature information, a first image in which areas of the pathological slide image are classified by type, by using the first machine learning model. For example, the processor 310 may classify the pathological slide image into at least one of a cancer area, a cancer stroma area, a necrosis area, and a background area and generate a first image showing the result of classification. The example in which the processor 310 classifies the pathological slide image into the at least one area is as described above.

As another example, the processor 310 may generate feature information by analyzing, in a sliding window method, a pathological slide image magnified at a third magnification. The example in which the processor 310 generates the feature information by using the sliding window method is described below with reference to FIG. 10.

As another example, the feature information generated by the processor 310 may include at least one of a cancer area, a tumor cell density, and information regarding a biomarker score. The above-described example in which the processor 310 generates the feature information is described below with reference to FIG. 12.

In operation 430, the processor 310 may detect at least one cell included in the at least one tissue from the pathological slide image, by using the pathological slide image and the feature information.

As an example, the processor 310 may detect the at least one cell from the pathological slide image by using a second machine learning model. Also, the processor 310 may process the result of detecting the at least one cell, on the basis of the result of classifying, by type, the areas within the pathological slide image, which are included in the feature information generated in operation 420. For example, the processor 310 may also detect a cell in a method of collectively excluding cells, which are not included in any type of area (e.g., a cancer area), from one type (e.g., a tumor cell). Meanwhile, the first machine learning model and the second machine learning model may be the same model or different models.

As another example, the processor 310 may detect, by using the second machine learning model, at least one cell on the basis of the pathological slide image and the first image. For example, the second machine learning model may be a machine learning model trained to detect a cell in a pathological slide image. Meanwhile, as described above, the first machine learning model and the second machine learning model may be the same model or different models.

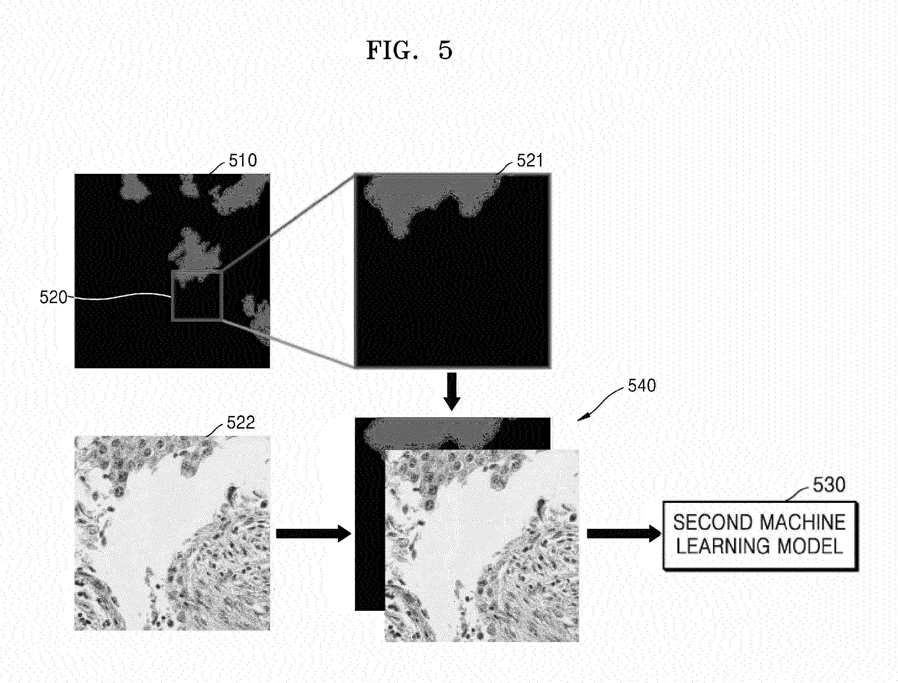

For example, the processor 310 may detect at least one cell from the pathological slide image on the basis of a third image in which the first image and a second image are merged. Here, the second image may refer to an image acquired from a portion corresponding to the first image within the pathological slide image. Hereinafter, the above-described example in which the processor 310 operates is described in detail with reference to FIG. 5.

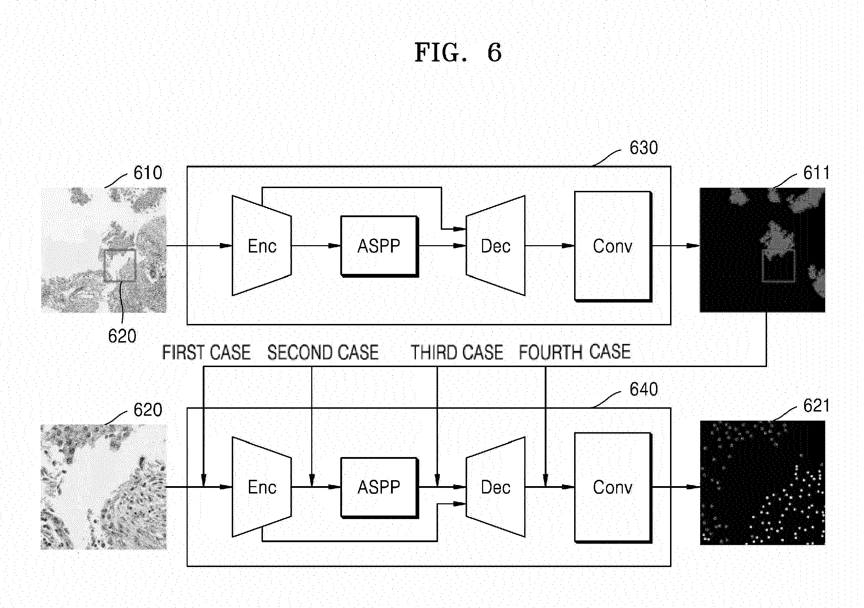

In some embodiments, the processor 310 may detect at least one cell from the pathological slide image by using the first image in at least one of intermediate operations of the second machine learning model that uses the pathological slide image as an input. Hereinafter, the above-described example in which the processor 310 operates is described in detail with reference to FIG. 6.

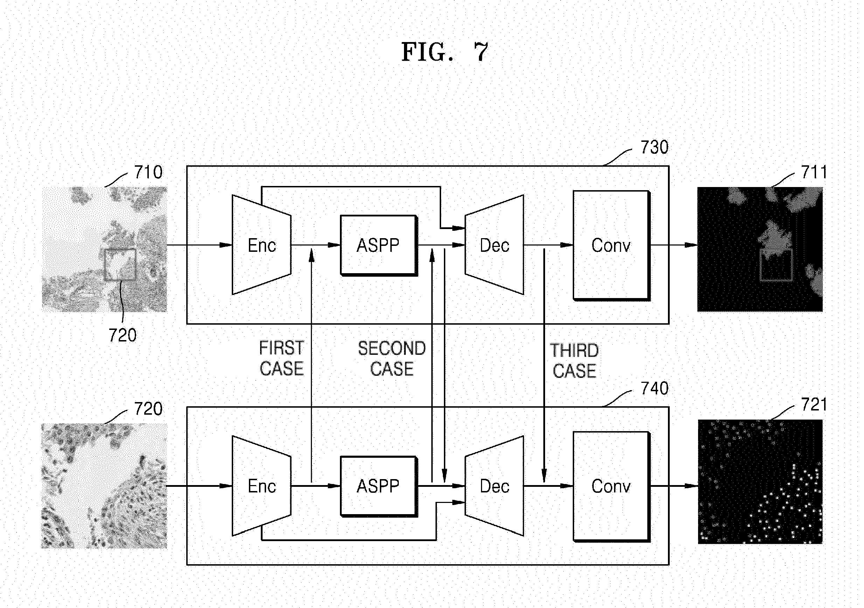

In some embodiments, the processor 310 may detect at least one cell from the pathological slide image by mutually using information generated from at least one of intermediate operations of the first machine learning model and information generated from at least one of intermediate operations of the second machine learning model in at least one of the intermediate operations of the first machine learning model and at least one of the intermediate operations of the second machine learning model. Hereinafter, the above-described example in which the processor 310 operates is described in detail with reference to FIG. 7.

FIG. 5 is a view illustrating an example in which a processor detects a cell from a pathological slide image, according to an embodiment.

FIG. 5 illustrates an example of a third image 540 in which an image 521, which is a portion 520 of a first image 510, and a second image 522 are merged. The first image 510 may be an image showing feature information generated from a pathological slide image. For example, the first image 510 may be an image in which tissues appearing in a whole portion or a portion of the pathological slide image are labeled by type. The second image 522 may be an image analyzed by a second machine learning model 530. For example, the second image 522 may be the whole pathological slide image or a portion of the pathological slide image.

As described above with reference to FIGS. 1 to 2B, a strong correlation may be present between cell and tissue classes. Therefore, when a tissue label for the pathological slide image is known in advance, accuracy of detecting a cell from the pathological slide image may be improved.

Meanwhile, the first image 510 and the second image 522 may be images showing the same subject, and the second image 522 may be an image corresponding to the portion 520 of the first image 510. For example, the second image 522 may be an image obtained by magnifying a portion of the pathological slide image corresponding to the portion 520 at a high magnification.

Referring to FIG. 5, the second image 522 may be an image corresponding to the portion 520 of the first image 510. For example, the portion 520 may be an image of an area from which a cell needs to be detected, and the area from which the cell needs to be detected may be set by a user or may be automatically set by the processor 310. In this case, the processor 310 may generate the third image 540 by merging the image 521 and the second image 522. For example, merging the image 521 and the second image 522 may indicate that the image 521 and the second image 522 overlap each other. For example, the processor 310 may generate the image 521 by selecting the portion 520 from the first image 510 and upsampling the portion 520. Accordingly, information regarding a tissue appearing in the image 521 may have a strong correlation with cells appearing in the second image 522. The processor 310 may generate the third image 540 by connecting the image 521 and the second image 522 on a channel basis.

Also, the processor 310 may detect at least one cell from the third image 540 by using the second machine learning model 530. Pieces of information regarding a tissue and a cell within the same area may be linked to each other in the third image 540, and thus, accuracy of cell detection by analysis of the third image 540 may be higher than accuracy of cell detection by analysis of the second image 522.

Meanwhile, FIG. 5 illustrates that the third image 540 is analyzed by the second machine learning model 530, but the disclosure is not limited thereto. In other words, the third image 540 may be analyzed by a first machine learning model, and the first machine learning model and the second machine learning model 530 may be the same model.

FIG. 6 is a view illustrating another example in which a processor detects a cell from a pathological slide image, according to an embodiment.

Referring to FIG. 6, feature information may be acquired from a pathological slide image 610 via a first machine learning model 630, and at least one cell may be detected from a second image 620 via a second machine learning model 640. For example, the first machine learning model 630 may generate, as feature information, a first image 611 in which tissues within the pathological slide image 610 are labeled by type. Also, the second image 620 may be a portion 620 of the pathological slide image 610 and may be an image showing a portion from which a cell needs to be detected. For example, the second image 620 may be an image showing a region of interest set by a user or the processor 310.

FIG. 6 illustrates that each of the first machine learning model 630 and the second machine learning model 640 includes an encoder Enc, a pooling module ASPP, a decoder Dec, and a convolution module Conv, but the disclosure is not limited thereto. Each of the first machine learning model 630 and the second machine learning model 640 may further include at least one layer needed for analyzing an image or may less include the at least one layer. Also, at least one of the encoder Enc, the pooling module ASPP, the decoder Dec, and the convolution module Conv may include at least one layer.

The processor 310 may use the first image 611 in an operation of the second machine learning model 640. For example, the processor 310 may use information (hereinafter, referred to as first information) regarding an area corresponding to the second image 620 from among the first image 611 in at least one of intermediate operations (e.g., a first case to a fourth case) of the second machine learning model 640.

Referring to FIG. 6, the processor 310 may inject the first information into an input (e.g., the first case) of the encoder Enc of the second machine learning model 640, an output (e.g., the second case) of the encoder Enc, an output (e.g., the third case) of the pooling module ASPP, and an output of the decoder Dec. Here, the pathological slide image 610 and the second image 620 may show different areas, and thus, the processor 310 may select the portion 620 from the first image 611, upsample the selected portion 620, and input the upsampled portion 620 into the second machine learning model 640 to match information between a feature map of a tissue and a feature map of a cell.

Accordingly, the second machine learning model 640 may accurately detect at least one cell from the second image 620. An image 621 shown in FIG. 6 may be an image showing a result of detecting a cell, and differently colored dots displayed in the image 621 may respectively refer to different types of cells (e.g., a tumor cell, a background cell, and the like).

FIG. 7 is a view illustrating another example in which a processor detects a cell from a pathological slide image, according to an embodiment.

Referring to FIG. 7, feature information may be acquired from a pathological slide image 710 via a first machine learning model 730, and at least one cell may be detected from a second image 720 via a second machine learning model 740. For example, the first machine learning model 730 may generate, as feature information, a first image 711 in which tissues within the pathological slide image 710 are labeled by type. Also, the second image 720 may be a portion 720 of the pathological slide image 710 and may be an image showing a portion from which a cell needs to be detected. For example, the second image 720 may be an image showing a region of interest set by a user.

The first machine learning model 730 and the second machine learning model 740 shown in FIG. 7 may have the same structures as the first machine learning model 630 and the second machine learning model 640 shown in FIG. 6. Accordingly, detailed descriptions of the structures of the first machine learning model 730 and the second machine learning model 740 are omitted.

The processor 310 may mutually use information (hereinafter, referred to as second information) generated from at least one of intermediate operations of the first machine learning model 730 and information (hereinafter, referred to as third information) generated from at least one of intermediate operations of the second machine learning model 740 in at least one of the intermediate operations of the first machine learning model 730 and at least one of the intermediate operations of the second machine learning model 740.

Referring to FIG. 7, the processor 310 may inject the third information into an output (e.g., a first case) of an encoder Enc of the first machine learning model 730. In addition, the processor 310 may inject the second information into an output (e.g., a second case) of an encoder Enc of the second machine learning model 740, and may inject the third information into the output (e.g., the second case) of the encoder Enc of the first machine learning model 730. Also, the processor 310 may inject the second information into an output (e.g., a third case) of a decoder Dec of the second machine learning model 740. In other words, the processor 310 may send a feature map of a cell to the first machine learning model 730 in a first phase and send a feature map of a tissue to the second machine learning model 740 in a last phase. Accordingly, finally, the processor 310 may accurately detect at least one cell from the second image 720. An image 721 shown in FIG. 7 may be an image showing a result of detecting a cell, and differently colored dots displayed in the image 721 may respectively refer to different types of cells (e.g., a tumor cell, a background cell, and the like).

Comparing the example shown in FIG. 7 to the example shown in FIG. 6, in the example of FIG. 6, the feature map of the tissue may be injected into the second machine learning model 640 in one direction, but in the example of FIG. 7, the feature map of the tissue and the feature map of the cell may be injected into the first machine learning model 730 and the second machine learning model 740 in both directions.

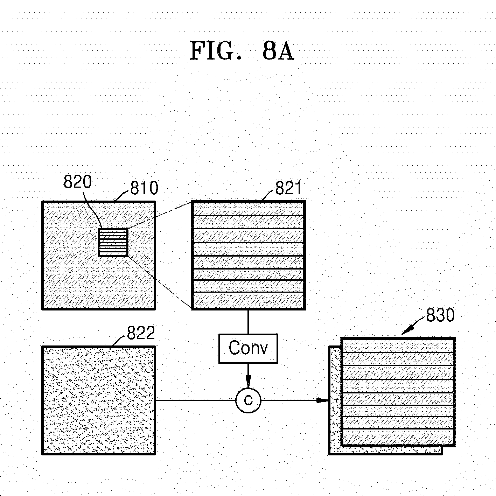

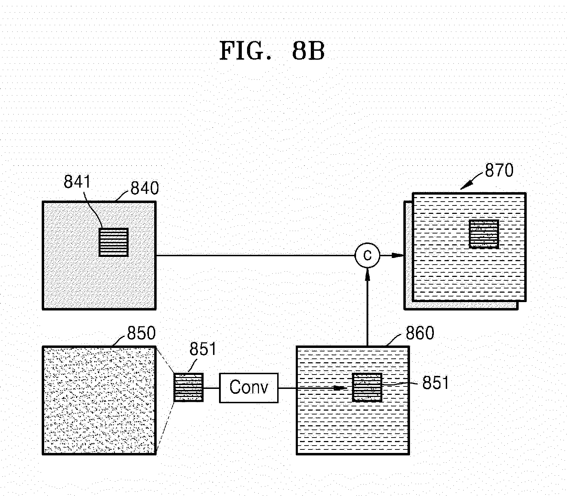

Meanwhile, according to the examples shown in FIGS. 6 and 7, the feature map of the tissue and the feature map of the cell may be shared among the machine learning models 630, 640, 730, and 740 or unilaterally. Therefore, the feature map of the tissue and the feature map of the cell need to be aligned with each other in the same dimension. The example in which the feature map of the tissue and the feature map of the cell are aligned in the same dimension is described with reference to FIGS. 8A and 8B.

FIGS. 8A and 8B are views illustrating an example in which a feature map of a tissue and a feature map of a cell are aligned in the same dimension, according to an embodiment.

Referring to FIGS. 8A and 8B, feature maps of tissues may be images 810 and 840 (i.e., first images) in which tissues are labeled in pathological slide images. Also, feature maps of cells may be images 822 and 850 in which cells extracted from second images are expressed. Accordingly, the images 810 and 840 and the images 822 and 850 may show different fields of view. For example, magnifications of the images 810 and 840 may be relatively lower than magnifications of the images 822 and 850.

FIG. 8A illustrates an example of manipulating a portion 820 of the image 810 to align the portion 820 with the image 822 (i.e., align a cell in a tissue), and FIG. 8B illustrates an example of manipulating the image 850 to align the image 850 with image 840 (i.e., align a tissue in a cell).

Referring to FIG. 8A, the processor 310 may generate an image 821 by selecting the portion 820 from the image 810 by considering the image 822, and upsampling the portion 820. Accordingly, the image 821 and the image 822 may be images showing the same field of view (i.e., the same magnification). Subsequently, the processor 310 may generate an image 830 by merging the image 821 and the image 822. For example, merging the image 821 and the image 822 may indicate that the image 821 and the image 822 overlap each other. Accordingly, the image 830 may show a result of a feature map of a tissue and a feature map of a cell being aligned in the same dimension.

Referring to FIG. 8B, the processor 310 may generate an image 851 by downsampling the image 850, and may generate an image 860 by performing zero padding on the image 851 by considering a location of a portion 841 within the image 840. Accordingly, the image 860 may be an image showing the same field of view (i.e., the same magnification) as the image 840. Subsequently, the processor 310 may generate an image 870 by merging the image 840 and the image 860. For example, the processor 310 may generate the image 870 by overlapping the image 840 and the image 860 with each other. Accordingly, the image 870 may show a result of a feature map of a tissue and a feature map of a cell being aligned in the same dimension.

Meanwhile, at least one of the first machine learning models 630 and 730 and the second machine learning models 640 and 740 may be trained by data generated on the basis of at least one patch included in a pathological slide image.

Here, training data may include at least one of a first patch magnified at a first magnification, at least one annotated tissue based on the first patch, a second patch magnified at a second magnification, at least one annotated cell based on the second patch, and information regarding a positional relationship between the first patch and the second patch.

As an example, the training data may include all of the first patch magnified at the first magnification, the at least one annotated tissue based on the first patch, the second patch magnified at the second magnification, the at least one annotated cell based on the second patch, and the information regarding the positional relationship between the first patch and the second patch. For example, training data D may be expressed as a data set below.

$$D = \{(x_s, y_s^c, x_l, y_l^t, c_x, c_y)_i\}_{i=1}^{N}$$

Here, $x_l$ may refer to the first patch magnified at the first magnification, and $x_s$ may refer to the second patch magnified at the second magnification. Here, the first magnification may be a magnification relatively less than the second magnification. Also, $y_i^t$ may refer to the at least one annotated tissue based on the first patch, and $y_s^c$ may refer to the at least one annotated cell based on the second patch. In addition, $c_x$ and $c_y$ may refer to the information regarding the positional relationship between the first patch $x_l$ and the second patch $x_s$. In other words, Cx and Cy may refer to relative coordinates of a region center at which the second patch $x_s$ is aligned within the first patch $x_l$.

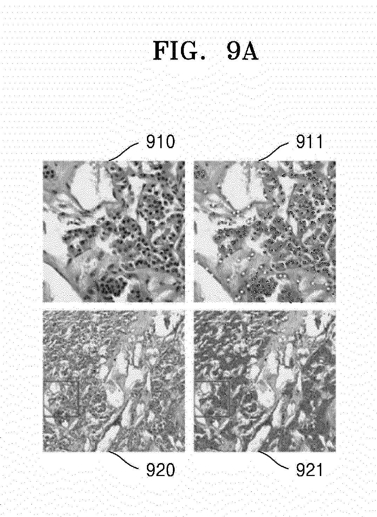

As another example, the training data may not include some of the first patch magnified at the first magnification, the at least one annotated tissue based on the first patch, the second patch magnified at the second magnification, the at least one annotated cell based on the second patch, and the information regarding the positional relationship between the first patch and the second patch. For example, the training data may not include at least one of the at least one annotated tissue based on the first patch and the at least one annotated cell based on the second patch. Hereinafter, examples of training data are described with reference to FIGS. 9A to 9C.

FIGS. 9A to 9C are views illustrating examples of training data of a first machine learning model and/or a second machine learning model, according to an embodiment.

Referring to FIG. 9A, training data may include a first patch 920, a second patch 910, an image 921 showing at least one annotated tissue based on the first patch 920, and an image 911 showing at least one annotated cell based on the second patch 910. Meanwhile, although not shown in FIGS. 9A to 9C, the training data may further include information regarding relative positional relationships between first patches 920, 940, and 960 and second patches 910, 930, and 950.

As described above with reference to FIGS. 5 to 8, an image (e.g., a first image) including an annotated tissue and a pathological slide image (or a partial image of the pathological slide image) may be merged, and the merged image may be input into a machine learning model. Here, the merged image may include an image in which the images described above overlap each other. Therefore, to train a machine learning model or perform an inference, data input into the machine learning model may include not only the first patch 920 and the second patch 910, but also the image 921 annotated with a tissue and an image 911 annotated with a cell.

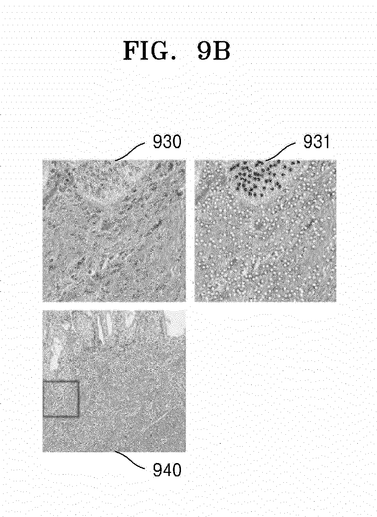

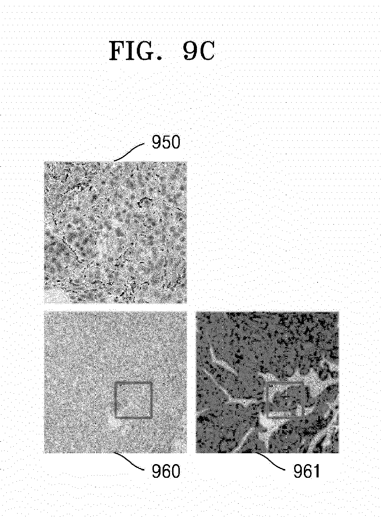

However, the training data may not include any one of the image 921 and the image 911. As an example, referring to FIG. 9B, training data may include the first patch 940, the second patch 930, and an image 931 showing at least one annotated cell based on the second patch 930. As another example, referring to FIG. 9C, training data may include the first patch 960, the second patch 950, and an image 961 showing at least one annotated tissue based on the first patch 960.

In summary, some of training data used to train a first machine learning model and/or a second machine learning model may be training data including only any one of annotation information of a tissue and annotation information of a cell in a pathological slide image.

Meanwhile, the processor 310 may detect a cell in a method of classifying areas by type in the pathological slide image and collectively excluding cells, which are not included in a certain type of area (e.g., a cancer area), from one type (e.g., a tumor cell). In this case, an error such as incorrectly detecting a cell outside the cancer area as a tumor cell may be reduced. Hereinafter, the above-described example in which the processor 310 detects a cell is described with reference to FIG. 10.

FIG. 10 is a view illustrating another example in which a processor detects a cell from a pathological slide image, according to an embodiment.

Referring to FIG. 10, the processor 310 may detect a cell from a pathological slide image 1010 by using a sliding window method.

The processor 310 may generate feature information 1030 by analyzing, in a sliding window method, a pathological slide image 1020 magnified at a third magnification. In detail, the processor 310 may generate the feature information 1030 by magnifying the pathological slide image 1010 at the third magnification and sliding a window 1021 having a certain size on the magnified pathological slide image 1020. Here, the third magnification may be a magnification showing a wide field of view and refer to a magnification lower than a fourth magnification described below.

For example, the processor 310 may detect a tissue (i.e., classify the tissue by type) from the magnified pathological slide image 1020 in the sliding window method described above. Here, the processor 310 may perform interpolation by merging predicted values of areas overlapping according to the sliding of the window 1021.

Also, the processor 310 may analyze, in a sliding window method, a pathological slide image 1040 magnified at the fourth magnification and extract cells from the magnified pathological slide image 1040. In detail, the processor 310 may generate feature information by magnifying the pathological slide image 1010 at the fourth magnification and sliding a window 1041 having a certain size on the magnified pathological slide image 1040. Here, the fourth magnification may be a magnification showing a narrow field of view and refer to a magnification higher than the third magnification described above.

Here, a "magnification" may have a meaning corresponding to a "field of view (FOV)". For example, an operation in which the processor 310 magnifies the pathological slide image 1010 at each of the third magnification and the fourth magnification and extracts some images by using windows having the same size (here, the fourth magnification is a magnification higher than the third magnification) and an operation in which the processor 310 extracts some images from the pathological slide image 1010 with a third FoV and a fourth FoV, respectively (here, the fourth FoV is less than the third FoV), may refer to the same operation.

Also, the processor 310 may detect at least one cell by using a result 1050 of extracting the cells and the feature information 1030. For example, the processor 310 may determine that cells present in an area other than a cancer area in the pathological slide image 1040 are collectively not tumor cells, by using information regarding the cancer area, which is included in the feature information 1030.

Meanwhile, the processor 310 may increase accuracy of cell detection by differently setting a magnification of an image used for training of a machine learning model that detects a cell from a pathological slide image and a magnification of an image used for an inference. Hereinafter, the above-described example in which the processor 310 detects a cell is described with reference to FIG. 11.

FIG. 11 is a view illustrating another example in which a processor detects a cell from a pathological slide image, according to an embodiment.

Referring to FIG. 11, the processor 310 may detect a cell from a pathological slide image 1110 by using a third machine learning model 1150. Here, a third machine learning model 1140 may be trained through a training process 1160, and the trained third machine learning model 1150 may be used in an inference process 1170.

Here, a magnification of an image 1120 used in the training process 1160 for the third machine learning model 1140 and a magnification of an image 1130 used in the inference process 1170 via the third machine learning model 1150 may be different from each other. For example, the image 1120 used in the training process 1160 may be an image having a narrow field of view (e.g., a 1024 * 1024 size with a resolution of 0.193 MPP), and the image 1130 used in the inference process 1170 1130 may be an image having a wide field of view (e.g., a 2048 * 2048 size with a resolution of 0.193 MPP). Accordingly, the third machine learning model 1150 may more easily compare a difference between a size of a nucleus and a size of a cell over a wide area of the pathological slide image 1110. Therefore, accuracy of an inference by the third machine learning model 1150 may be increased, and a possibility that the third machine learning model 1150 confuses and determines a type of cell may be reduced.

In other words, a field of view of the image 1130 input during the inference via the third machine learning model 1150 may be set to be greater than a field of view of the image 1120 used during training of the third machine learning model 1140, and thus, accuracy of the inference may be increased. Here, a difference between the field of view of the image 1120 and the field of view of the image 1130 may be set variously or flexibly according to types of images used in the processes described above with reference to FIG. 11.

As described above with reference to FIGS. 4 to 11, the processor 310 may use whole context of a pathological slide image to detect a cell from the pathological slide image. In other words, to generate a final result (i.e., to detect a cell from the pathological slide image), the processor 310 may use, as the whole context, information considered in an intermediate process (e.g., information regarding which area is a cancer area).

Meanwhile, the processor 310 may not only detect at least one cell from the pathological slide image, but also further detect at least one of biomarker information, immune phenotype information, lesion information, genomic mutation information, and genomic signature information which are expressed on at least one tissue. Here, the processor 310 may also use at least one of a cancer area, a tumor cell density, and information regarding a biomarker score as the feature information (i.e., the whole context). Here, the biomarker score may include quantitative information related to a CPS score, a TPS score, an HER2 score, and PD-L1, and the like. Hereinafter, the above-described example of the operation of the processor 310 is described with reference to FIG. 12.

FIG. 12 is a view illustrating an example in which a processor predicts various types of information from a pathological slide image, according to an embodiment.

Referring to FIG. 12, the processor 310 may consider a first feature 1210 to a sixth feature 1260 to derive a seventh feature 1270 from a pathological slide image. For example, the seventh feature 1270 may include at least one of biomarker information, immune phenotype information, lesion information, genomic mutation information, and genomic signature information which are expressed on at least one tissue. In addition, the first feature 1210 to the sixth feature 1260 may be a cancer area, a tumor cell density, and information regarding a biomarker score (e.g., quantitative information related to a CPS score, a TPS score, an HER2 score, and PD-L1, and the like).

In a process of predicting the seventh feature 1270 from the pathological slide image by using a machine learning model, the processor 310 may consider various types of feature information (e.g., the first feature 1210 to the sixth feature 1260) of the pathological slide image. Here, the processor 310 may determine a desirable order or priority in which features (e.g., the first feature 1210 to the sixth feature 1260) need to be considered. Accordingly, the processor 310 may predict the features (e.g., the first feature 1210 to the sixth feature 1260) according to the desirable order or priority, and predict the seventh feature 1270 by using the predicted features (e.g., the first feature 1210 to the sixth feature 1260).

Here, the desirable order or priority may be preset by a user, and an optimized hierarchy among the features (e.g., the first feature 1210 to the sixth feature 1260) may be generated by a machine learning model. Meanwhile, for convenience of description, FIG. 12 illustrates that six pieces of feature information are considered to generate the seventh feature 1270, but the number of pieces of feature information is not limited to the above-mentioned number.

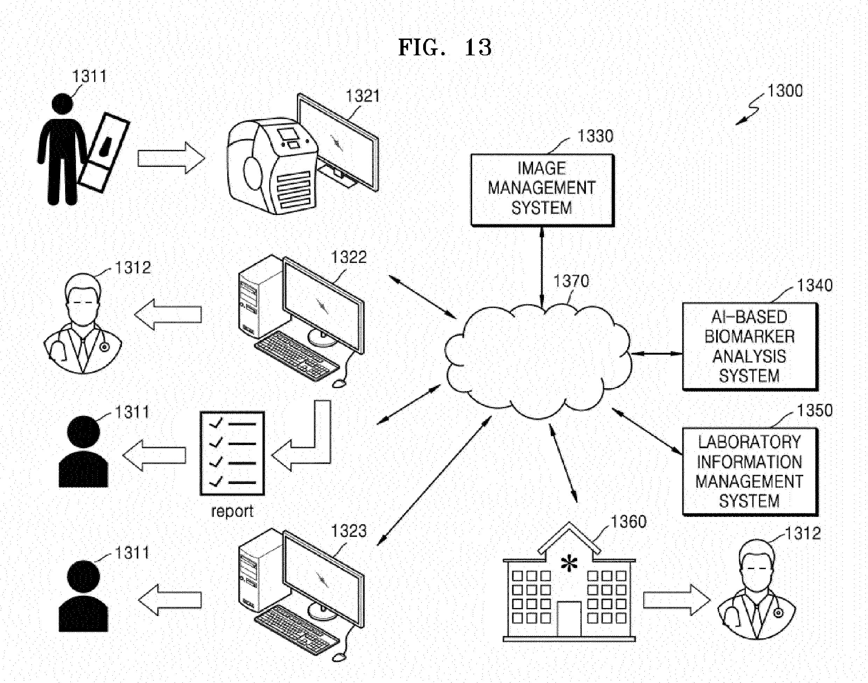

FIG. 13 is a view illustrating an example of a system for analyzing a pathological slide image.

FIG. 13 illustrates an example of a system 1300 and a network for providing, processing, and reviewing slide images of tissue specimens by using a machine learning model.

According to various embodiments, the method described above with reference to FIGS. 1 to 12 may be performed by at least one of user terminals 1322 and 1323, an image management system 1330, an AI-based biomarker analysis system 1340, a laboratory information management system 1350, and a hospital or research room server 1360, or a combination thereof.

A scanner 1321 may acquire a digitized image from a tissue sample slide generated by using a tissue sample of a subject 1311. For example, each of the scanner 1321, the user terminals 1322 and 1323, the image management system 1330, the AI-based biomarker analysis system 1340, the laboratory information management system 1350, and/or the hospital or research room server 1360 may be connected to a network 1370, such as the Internet, through one or more computers, servers, and/or mobile devices, or may communicate with a user 1312 through one or more computers, and/or mobile devices.

The user terminals 1322 and 1323, the image management system 1330, the AI-based biomarker analysis system 1340, the laboratory information management system 1350, and/or the hospital or research room server 1360 may generate or otherwise may acquire, from another device, tissue samples of one or more subjects 1311, tissue sample slides (e.g., pathological slides), digitized images of the tissue sample slides (e.g., the pathological slides), or any combination thereof. In addition, the user terminals 1322 and 1323, the image management system 1330, the AI-based biomarker analysis system 1340, the laboratory information management system 1350, and/or the hospital or research room server 1360 may acquire any combination of subject-specific information, such as age, medical history, cancer treatment history, family history, and past biopsy records of the subject 1311, or disease information of the subject 1311.

The scanner 1321, the user terminals 1322 and 1323, the AI-based biomarker analysis system 1340, the laboratory information management system 1350, and/or the hospital or research room server 1360 may transmit the digitized slide images, the subject-specific information, and/or results of analyzing the digitized slide images to the image management system 1330 via the network 1370. The image management system 1330 may include a storage for storing received images and a storage for storing analysis results.

Also, according to various embodiments, a machine learning model, which is learned and trained to predict, from a slide image of the subject 1311, at least one of information regarding at least one cell, information regarding at least one area, information related to a biomarker, medical diagnosis information, and/or medical treatment information, may be stored and operated in the user terminals 1322 and 1323, the image management system 1330, and the like.

According to the above description, the processor 310 may infer various types of information from a pathological slide image with high accuracy by using a machine learning model.

Meanwhile, the above-described method may be written as a program that may be executed on a computer, and may be implemented in a general-purpose digital computer that operates the program by using a computer-readable recording medium. In addition, a structure of data used in the above-described method may be recorded on a computer-readable recording medium via various means. The computer-readable recording medium may include storage media such as magnetic storage media (e.g., ROM, RAM, a USB, a floppy disk, a hard disk, and the like) and optical reading media (e.g., CD-ROM, DVD, and the like).

One of ordinary skill in the art related to the present embodiment will understand that the present embodiment may be implemented in a modified form within the scope that does not depart from the essential characteristics of the above description. Therefore, the disclosed methods should be considered in an illustrative sense rather than a restrictive sense, and the scope of the disclosure should defined by claims rather than by the foregoing description and should be construed as including all differences falling within the scope equivalent thereto.

**Claims**

1.  A computing apparatus comprising:

    at least one memory; and
    at least one processor, wherein the processor is configured to: acquire a pathological slide image showing at least one tissue; generate feature information related to at least one area of the pathological slide image; and detect, from the pathological slide, at least one cell included in the at least one tissue image by using the pathological slide image and the feature information.

2.  The computing apparatus of claim 1, wherein the processor is further configured to:

    generate, as the feature information, a first image showing a first type of area of the pathological slide image by using a first machine learning model;
    detect a second type of cells from the pathological slide image by using a second machine learning model; and
exclude, from a detection result, at least one cell, which is not included in the first type of area, from among the detected cells.

3. The computing apparatus of claim 1, wherein the processor is further configured to:

generate, as the feature information, a first image in which areas of the pathological slide image are classified by type, by using a first machine learning model; and
detect, by using a second machine learning model, the at least one cell on the basis of the pathological slide image and the first image, wherein
the type includes at least one of a cancer area, a cancer stroma area, a necrosis area, and a background area.

4. The computing apparatus of claim 3, wherein the processor is further configured to detect the at least one cell on the basis of a third image in which the first image is merged with a second image acquired from a portion corresponding to the first image within the pathological slide image.

5. The computing apparatus of claim 3, wherein the processor is further configured to detect the at least one cell by using the first image in at least one of intermediate operations of the second machine learning model that uses the pathological slide image as an input.

6. The computing apparatus of claim 3, wherein the processor is further configured to detect the at least one cell by mutually using information generated from at least one of intermediate operations of the first machine learning model and information generated from at least one of intermediate operations of the second machine learning model in at least one of the intermediate operations of the first machine learning model and at least one of the intermediate operations of the second machine learning model.

7. The computing apparatus of claim 3, wherein at least one of the first machine learning model and the second machine learning model is trained by data generated on the basis of at least one patch included in the pathological slide image, and the generated data includes at least one of a first patch magnified at a first magnification, at least one annotated tissue based on the first patch, a second patch magnified at a second magnification, at least one annotated cell based on the second patch, and information regarding a positional relationship between the first patch and the second patch.

8. The computing apparatus of claim 1, wherein the processor is further configured to: generate the feature information by analyzing, in a sliding window method, the pathological slide image magnified at a third magnification; extract cells from the pathological slide image by analyzing, in the sliding window method, the pathological slide image magnified at a fourth magnification; and detect the at least one cell by using a result of extracting the cells and the feature information.

9. The computing apparatus of claim 1, wherein the processor is further configured to detect the at least one cell from the pathological slide image by using a third machine learning model, and a magnification of an image used for training of the third machine learning model and a magnification of an image used for an inference by the third machine learning model are different from each other.

10. The computing apparatus of claim 1, wherein the feature information includes at least one of a cancer area, a tumor cell density, and information regarding a biomarker score, and the processor is further configured to further detect at least one of biomarker information, immune phenotype information, lesion information, genomic mutation information, and genomic signature information which are expressed on the at least one tissue.

11. A method of analyzing a pathological slide image, the method comprising:

acquiring a pathological slide image showing at least one tissue;
generating feature information related to at least one area of the pathological slide image; and
detecting, from the pathological slide, at least one cell included in the at least one tissue image by using the pathological slide image and the feature information.

12. The method of claim 11, wherein the generating includes generating, as the feature information, a first image showing a first type of area of the pathological slide image by using a first machine learning model, and the detecting includes: detecting a second type of cells from the pathological slide image by using a second machine learning model; and excluding, from a detection result, at least one cell, which is not included in the first type of area, from among the detected cells.

13. The method of claim 11, wherein the generating includes

generating, as the feature information, a first image in which areas of the pathological slide image are classified by type, by using a first machine learning model, and

the detecting includes detecting, by using a second machine learning model, the at least one cell on the basis of the pathological slide and the first image, and the type includes at least one of a cancer area, a cancer stroma area, a necrosis area, and a background area.

14. The method of claim 11, wherein the generating includes generating the feature information by analyzing, in a sliding window method, the pathological slide image magnified at a third magnification, and the detecting includes: extracting cells from the pathological slide image by analyzing, in the sliding window method, the pathological slide image magnified at a fourth magnification; and detecting the at least one cell by using a result of extracting the cells and the feature information.

15. A computer-readable recording medium having recorded thereon a program for causing a computer to execute the method of claim 11.

# FIG. 1

FIG. 2A

FIG. 2B

# FIG. 3

# FIG. 4

```
           ┌─────────┐
           │  START  │
           └────┬────┘
                ▼
┌───────────────────────────────────┐
│    ACQUIRE PATHOLOGICAL SLIDE IMAGE │─── 410
│     SHOWING AT LEAST ONE TISSUE     │
└───────────────┬───────────────────┘
                ▼
┌───────────────────────────────────┐
│  GENERATE FEATURE INFORMATION RELATED │─── 420
│   TO AT LEAST ONE AREA OF PATHOLOGICAL│
│             SLIDE IMAGE              │
└───────────────┬───────────────────┘
                ▼
┌───────────────────────────────────┐
│  DETECT, FROM PATHOLOGICAL SLIDE IMAGE,│
│  AT LEAST ONE CELL  INCLUDED IN AT LEAST│─── 430
│  ONE TISSUE BY USING PATHOLOGICAL SLIDE │
│   IMAGE AND FEATURE INFORMATION      │
└───────────────┬───────────────────┘
                ▼
           ┌─────────┐
           │   END   │
           └─────────┘
```

# FIG. 5

EP 4 369 354 A1

## FIG. 6

FIRST CASE    SECOND CASE    THIRD CASE    FOURTH CASE

# FIG. 7

# FIG. 8A

# FIG. 8B

# FIG. 9A

FIG. 9B

# FIG. 9C

# FIG. 10

# FIG. 11

TRAINING PROCESS (1160)

THIRD MACHINE LEARNING MODEL (1140)

THIRD MACHINE LEARNING MODEL (1150)

INFERENCE PROCESS (1170)

FIG. 12

EP 4 369 354 A1

FIG. 13

EP 4 369 354 A1

Europäisches
Patentamt

European
Patent Office

Office européen
des brevets

# EUROPEAN SEARCH REPORT

**Application Number**

EP 23 20 9046

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/258223 A1 (YIP STEPHEN [US] ET AL) 13 August 2020 (2020-08-13) | 1-5,7-15 | INV.<br>G16H30/40 |
| A | * paragraphs [0013], [0016] – [0020], [0090] – [0094], [0117], [0132] – [0188], [0208], [0209]; figures 1,7 * | 6 | G06T7/00<br>G06V10/82<br>G06V20/69<br>G16H50/20 |
| | ----- | | |
| X | KR 2021 0136885 A (YOO DONG GEUN ET AL) 17 November 2021 (2021-11-17) | 1-5,7-15 | |
| A | * paragraphs [0011], [0012], [0089], [0139], [0162] * | 6 | |
| | ----- | | |
| X | US 2022/036971 A1 (YOO DONGGEUN [KR] ET AL) 3 February 2022 (2022-02-03) | 1-5,7-15 | |
| A | * the whole document * | 6 | |
| | ----- | | |
| X,P | EP 4 246 525 A1 (LUNIT INC [KR]) 20 September 2023 (2023-09-20) | 1-5,7-15 | |
| A,P | * the whole document * | 6 | |
| | ----- | | |
| X | US 2022/028068 A1 (COSATTO ERIC [US] ET AL) 27 January 2022 (2022-01-27) | 1-5,7-15 | TECHNICAL FIELDS SEARCHED (IPC) |
| A | * the whole document * | 6 | G16H<br>G06T<br>G06V |
| | ----- | | |

The present search report has been drawn up for all claims

| Place of search | Date of completion of the search | Examiner |
|---|---|---|
| Munich | 4 March 2024 | Huber, Alexander |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

      ..................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 20 9046

This annex lists the patent family members relating to the patent documents cited in the above-mentioned European search report.
The members are as contained in the European Patent Office EDP file on
The European Patent Office is in no way liable for these particulars which are merely given for the purpose of information.

04-03-2024

| Patent document cited in search report | | | Publication date | Patent family member(s) | | | Publication date |
|---|---|---|---|---|---|---|---|
| US 2020258223 | A1 | | 13-08-2020 | US | 2020258223 | A1 | 13-08-2020 |
| | | | | US | 2021233238 | A1 | 29-07-2021 |
| | | | | US | 2021256690 | A1 | 19-08-2021 |
| | | | | US | 2022101519 | A1 | 31-03-2022 |
| | | | | US | 2022405919 | A1 | 22-12-2022 |
| | | | | US | 2023230195 | A1 | 20-07-2023 |
| KR 20210136885 | A | | 17-11-2021 | CN | 115298749 | A | 04-11-2022 |
| | | | | EP | 4105943 | A1 | 21-12-2022 |
| | | | | EP | 4148746 | A1 | 15-03-2023 |
| | | | | KR | 20210136884 | A | 17-11-2021 |
| | | | | KR | 20210136885 | A | 17-11-2021 |
| US 2022036971 | A1 | | 03-02-2022 | JP | 2023523882 | A | 08-06-2023 |
| | | | | KR | 20240015696 | A | 05-02-2024 |
| | | | | US | 2022036971 | A1 | 03-02-2022 |
| | | | | US | 2023420072 | A1 | 28-12-2023 |
| | | | | WO | 2021225421 | A1 | 11-11-2021 |
| EP 4246525 | A1 | | 20-09-2023 | EP | 4246525 | A1 | 20-09-2023 |
| | | | | JP | 2023138488 | A | 02-10-2023 |
| | | | | US | 2023298171 | A1 | 21-09-2023 |
| US 2022028068 | A1 | | 27-01-2022 | US | 2022028068 | A1 | 27-01-2022 |
| | | | | WO | 2022020471 | A1 | 27-01-2022 |

EPO FORM P0459

For more details about this annex : see Official Journal of the European Patent Office, No. 12/82